# EUROPÄISCHE PATENTSCHRIFT

(11) **EP 3 861 953 B1**
(45) Veröffentlichungstag und Bekanntmachung des Hinweises auf die Patenterteilung: **02.12.2026**
(21) Anmeldenummer: 21151963.2
(22) Anmeldetag: 17.01.2021
(51) Int. Cl.: A61B 18/12, A61B 18/14, A61B 90/90, A61B 90/98, A61B 17/32, A61B 18/00, A61B 90/00

(54) **ELEKTROCHIRURGISCHES SYSTEM, ELEKTROCHIRURGISCHES INSTRUMENT, UND VERFAHREN ZUM AUSLESEN VON KONFIGURATIONSDATEN**
ELECTROSURGICAL SYSTEM, ELECTROSURGICAL INSTRUMENT, AND CONFIGURATION DATA READOUT METHOD
SYSTÈME ÉLECTROCHIRUGICAL, INSTRUMENT ÉLECTROCHIRUGICAL, ET PROCÉDÉ DE LECTURE DES DONNÉES DE CONFIGURATION

(30) Priorität: 10.02.2020 DE 102020103280
(43) Veröffentlichungstag der Anmeldung: 11.08.2021
(73) Patentinhaber: OLYMPUS WINTER & IBE GMBH, 22045 Hamburg (DE)
(72) Erfinder: Kwik, Anne, 14165 Berlin (DE); Kersten, Lutz, 12163 Berlin (DE); Vahldiek, Thilo, 14467 Potsdam (DE); Schröder, Christian, 10713 Berlin (DE)
(74) Vertreter: Noack, Andreas

(56) Entgegenhaltungen:
- EP-A1- 3 506 278
- WO-A1-02/41798
- DE-A1- 102013 202 526
- DE-A1- 102015 201 470
- US-A1- 2016 220 299

## Beschreibung

Die Erfindung betrifft ein Elektrochirurgisches System, umfassend ein elektrochirurgisches Versorgungsgerät, und ein elektrochirurgisches Instrument, wobei das elektrochirurgische Instrument ein Speicherelement umfasst, welches durch das elektrochirurgische Versorgungsgerät ausgelesen werden kann, wenn das elektrochirurgische Instrument mit dem elektrochirurgischen Versorgungsgerät verbunden ist, und auf dem Speicherelement Konfigurationsdaten hinterlegt sind, welche von dem elektrochirurgischen Versorgungsgerät ausgewertet werden können, um mit dem elektrochirurgischen Instrument kompatible Betriebsparameter einzustellen. Ferner betrifft die Erfindung ein elektrochirurgisches Instrument eines entsprechenden elektrochirurgischen Systems, ein Verfahren zum Auslesen von Konfigurationsdaten, sowie ein elektrochirurgisches Versorgungsgerät. Elektrochirurgische Systeme werden in der Chirurgie seit längerem eingesetzt, um diverse Prozeduren durchzuführen. Bei der Elektrochirurgie im engeren Sinne wird zu behandelndes Gewebe direkt elektrischen Strömen ausgesetzt, bei denen es sich in der Regel um hochfrequente Wechselströme handelt. Durch entsprechende Dimensionierung der Instrumente und der verwendeten Ströme und Spannungen lassen sich unterschiedliche Gewebeeffekte erreichen, beispielsweise ein Koagulieren oder Schneiden des Gewebes. Um einen gewünschten Gewebeeffekt sicher zu erreichen ist es dabei erforderlich, dass ein von einem elektrochirurgischen Versorgungsgerät, beispielsweise einem Hochfrequenzgenerator, abgegebenes elektrochirurgisches Signal korrekt auf ein zur Anwendung kommendes elektrochirurgisches Instrument abgestimmt ist. Andernfalls kann es zu einem unzureichenden Gewebeeffekt oder gar zu einer Gefährdung des Patienten oder des behandelnden Arztes kommen.

Es sind auch Systeme bekannt, bei welchen ein zu behandelndes Gewebe zusätzlich oder ausschließlich über eine Ultraschall-Sonotrode beaufschlagt wird. Hier befindet sich zumeist in dem anzuwendenden Instrument ein Transducer, welcher ein von einem Versorgungsgerät in Form eines Ultraschallgenerator bereitgestelltes elektrisches Signal in Ultraschallschwingungen der Sonotrode umwandelt. Solche Ultraschallsysteme werden im Sinne der Erfindung ebenfalls als elektrochirurgische Systems angesehen. Auch bei entsprechenden Systemen ist es wichtig, dass das von dem Versorgungsgerät abgegebene elektrische Signal auf das Instrument abgestimmt ist, um eine korrekte Funktion des Instruments zu gewährleisten.

Bei modernen elektrochirurgischen Systemen ist das Instrument mit einem Speicherelement ausgestattet, auf welchem Konfigurationsdaten für das Versorgungsgerät hinterlegt sind.

Wird das Instrument mit dem Versorgungsgerät verbunden, so liest das Versorgungsgerät die Konfigurationsdaten aus dem Speicherelement aus und stellt das abgegebene elektrische Signal entsprechend ein. Dabei können zusätzliche Benutzereinstellungen an dem Versorgungsgerät möglich oder erforderlich sein. Entsprechende elektrochirurgische Systeme sind aus der US 2016/220299 A1 und der WO 02/41798 A1 bekannt.

Bei der ständigen Weiterentwicklung von elektrochirurgischen Systemen tritt der Effekt auf, dass die Entwicklung von Versorgungsgeräten einerseits und Instrumenten andererseits unterschiedlichen Entwicklungszyklen folgt. Dabei sollen neu entwickelte Instrumente den vollen Funktionsumfang neuerer Versorgungsgeräte ausnutzen können, gleichzeitig aber auch mit Versorgungsgeräten älterer Bauart betrieben werden können.

Für die Ausgestaltung der Konfigurationsdaten ergibt sich dabei das Problem, dass eine vollständige Abwärtskompatibilität, also die Kompatibilität mit Versorgungsgeräten älterer Bauart, in der Regel nur mit Einbußen bei der vollen Ausnutzung neuer verfügbarer Funktionalitäten erreichbar ist.

Eine Lösung dieses Problems wird in EP 3506278 A1 offengelegt, wobei zusätzliche Datenkreise die Rückwärtskompatibilität sicherstellen. Die zweiteilige Form von Anspruch 1 basiert auf diesem Dokument.

Es besteht daher eine Aufgabe der Erfindung darin, ein elektrochirurgisches System bereitzustellen, welches hinsichtlich der beschriebenen Problematik verbessert ist.

Diese Aufgabe wird gemäß eines ersten Aspekts der Erfindung gelöst durch ein Elektrochirurgisches System, umfassend: ein elektrochirurgisches Versorgungsgerät, und ein elektrochirurgisches Instrument, wobei das elektrochirurgische Instrument ein Speicherelement umfasst, welches durch das elektrochirurgische Versorgungsgerät ausgelesen werden kann, wenn das elektrochirurgische Instrument mit dem elektrochirurgischen Versorgungsgerät verbunden ist, und auf dem Speicherelement Konfigurationsdaten hinterlegt sind, welche von dem elektrochirurgischen Versorgungsgerät ausgewertet werden können, um mit dem elektrochirurgischen Instrument kompatible Betriebsparameter einzustellen, welches dadurch weitergebildet ist, dass die Konfigurationsdaten in einer flexiblen Datenstruktur organisiert sind, welche es erlaubt, auf dem Speicherelement gleichzeitig mehrere Sätze von Konfigurationsdaten vorzuhalten, welche von unterschiedlichen Typen von elektrochirurgischen Versorgungsgeräten ausgelesen und ausgewertet werden können.

Unterschiedliche Typen von elektrochirurgischen Versorgungsgeräten umfassen dabei unter anderem unterschiedliche Generationen von Versorgungsgeräten.

Durch die flexible Datenstruktur, in welcher die Konfigurationsdaten organisiert sind, eröffnet sich die Möglichkeit, einerseits einen Satz von Konfigurationsdaten vorzuhalten, welcher mit einem Versorgungsgerät einer älteren Generation kompatibel ist, und andererseits einen zweiten Satz von Konfigurationsdaten vorzuhalten, welcher mit einem Versorgungsgerät einer neueren, oder der neuesten, Generation kompatibel ist.

Der Begriff "flexible Datenstruktur" ist im Sinne der Erfindung so zu verstehen, dass Position, Aufbau, und/oder Länge der einzelnen Sätze von Konfigurationsdaten nicht fest vorgegeben sind, sondern insbesondere durch den Inhalt von Datenelementen der Konfigurationsdaten selbst bestimmt werden.

In einem elektrochirurgischen Systems gemäß der Erfindung umfasst die flexible Datenstruktur einen ersten Satz von Konfigurationsdaten, welcher an einer fest vorgegebenen Speicheradresse des Speicherelements abgelegt ist, und die flexible Datenstruktur umfasst weiterhin einen zweiten Satz von Konfigurationsdaten, welcher an einer zweiten Speicheradresse des Speicherelements abgelegt ist, welche von der Länge der ersten Satzes von Konfigurationsdaten abhängig ist. Mit einer entsprechenden Datenstruktur kann erreicht werden, dass ein Versorgungsgerät einer älteren Generation, welches nur einen einzelnen Satz von Konfigurationsdaten an einer vorgegebenen Adresse des Speicherelements erwartet, auf den ersten Satz von Konfigurationsdaten zugreifen kann. Der zweite Satz von Konfigurationsdaten ist demgemäß hinter dem ersten Satz von Konfigurationsdaten abgelegt und kann nur von Versorgungsgeräten neuerer Bauart ausgelesen werden, welche Sätze von Konfigurationsdaten von unterschiedlichen Adressen des Speicherelements lesen können.

Ein Satz von Konfigurationsdaten weist eine fest vorgegebene Länge auf. Dies kann vorzugsweise der erste Satz Konfigurationsdaten sein.

Wenigstens ein Satz von Konfigurationsdaten weist eine flexible Länge aufweisen, und umfasst ein Längendatenelement, welches die Länge des entsprechenden Satzes von Konfigurationsdaten anzeigt. Auf diese Weise ist es möglich, mehrere Sätze von Konfigurationsdaten quasi hintereinander im Speicherelement zu stapeln, wobei ein Versorgungsgerät die Adresse des nächsten Satzes von Konfigurationsdaten jeweils aus der Adresse des aktuellen Satzes und dem Inhalt des Längendatenelements bestimmen kann.

Das Längendatenelement selbst kann auch direkt die Adresse des nächsten Satzes von Konfigurationsdaten enthalten.

In einer vorteilhaften Weiterbildung eines elektrochirurgischen Systems nach der Erfindung kann die Datenstruktur ein Abschlussdatenelement umfassen, welches das Ende der Datenstruktur anzeigt. Es kann somit verhindert werden, dass das Versorgungsgerät versucht, auf einen nicht vorhandenen Satz von Konfigurationsdaten zuzugreifen. Hierbei könnten sonst Speicherzugriffsfehler auftreten, welche die Funktion des elektrochirurgischen Systems beeinträchtigen.

Die Datenstruktur kann für wenigstens einen Satz von Konfigurationsdaten einen betriebsmäßig nicht überschreibbaren Speicherbereich vorsehen.

Vorzugsweise kann die Datenstruktur für wenigstens einen Satz von Konfigurationsdaten einen beschreibbaren Speicherbereich vorsehen, und das elektrochirurgische Versorgungsgerät kann eingerichtet sein, Betriebsdaten in dem beschreibbaren Speicherbereich abzulegen.

Insbesondere bei elektrochirurgischen Instrumenten, die nur für eine begrenzte Anzahl von Anwendungen ausgelegt sind, kann beispielsweise die Anzahl der bereits durchgeführten Anwendungen, die abgegebene Energie/Leistung, Zeitstempel, oder ähnliche Informationen in dem beschreibbaren Speicherbereich abgelegt werden. Das Versorgungsgerät kann dann vor einer erneuten Aktivierung des Instruments diese Daten auslesen und entscheiden, ob die erneute Anwendung des Instruments zulässig ist.

Dabei kann der beschreibbare Speicherbereich zwei Betriebsdatenelemente umfassen, und das elektrochirurgische Versorgungsgerät kann eingerichtet sein, Betriebsdaten abwechselnd in eines der beiden Betriebsdatenelemente abzulegen. Hierdurch steht auch nach einem fehlerhaften Schreibvorgang, welcher beispielsweise durch elektrische Störsignale ausgelöst werden kann, jeweils noch der zuvor abgelegte Wert der Betriebsdaten zur Verfügung.

Die Aufgabe wird gemäß eines zweiten Aspekts der Erfindung gelöst durch ein elektrochirurgisches Instrument eines elektrochirurgischen Systems nach den obigen Ausführungen. Hinsichtlich der hierdurch erreichbaren Vorteile und Wirkungen wird auf die obigen Ausführungen explizit verwiesen.

Die Aufgabe wird gemäß eines dritten Aspekts der Erfindung gelöst durch ein Verfahren zum Auslesen von Konfigurationsdaten eines elektrochirurgischen Instruments in einem elektrochirurgischen System gemäß den obigen Ausführungen, mit den Schritten: a) Bestimmen einer Adresse, an welcher ein erster Satz Konfigurationsdaten hinterlegt ist, b) Einlesen eines Satzes von Konfigurationsdaten von der bestimmten Adresse, c) Ermitteln einer Adresse, an welcher ein nächster Satz von Konfigurationsdaten erwartet wird, d) Prüfen, ob an der in Schritt c) ermittelten Adresse ein Abschlussdatenelement abgelegt ist, und e) Wiederholen der Schritte b) bis d), bis an der ermittelten Adresse ein Abschlussdatenelement festgestellt wird.

Mittels des beschriebenen Verfahrens kann ein elektrochirurgisches Versorgungsgerät nacheinander alle in dem Speicherelement des elektrochirurgischen Instruments hinterlegten Sätze von Konfigurationsdaten auslesen. Das Versorgungsgerät kann dann einzelne, mehrere, oder alle Sätze von Konfigurationsdaten berücksichtigen, um das abzugebende elektrische Signal einzustellen.

In einer möglichen Weiterbildung eines Verfahren nach der Erfindung kann vor Schritt d) geprüft werden, ob an der ermittelten Adresse Daten hinterlegt sind, und das Verfahren kann beendet werden, wenn an der ermittelten Adresse keine Daten hinterlegt sind. Auf diese Weise können Probleme vermieden werden, die ggf. bei dem Auslesen von Konfigurationsdaten aus dem Speicherelement eines Instruments älterer Bauart entstehen können.

Weiterhin beschrieben wird ein elektrochirurgisches Versorgungsgerät eines elektrochirurgischen Systems, welches eingerichtet ist, ein Verfahren gemäß der obigen Angaben auszuführen.

Die Erfindung wird nachfolgend anhand einiger beispielhafter Darstellungen näher beschrieben, wobei die in der Darstellungen gezeigten Ausführungsbeispiele lediglich zum besseren Verständnis der Erfindung beitragen sollen, ohne diese einzuschränken.

Es zeigen:
- Fig. 1:: ein elektrochirurgisches System,
- Fig. 2:: eine Datenstruktur,
- Fig. 3:: einen Aufbau eines Satzes von Konfigurationsdaten,
- Fig. 4:: ein Verfahren zum Lesen und Schreiben von Betriebsdaten,
- Fig. 5:: ein Verfahren zum Auslesen von Konfigurationsdaten.

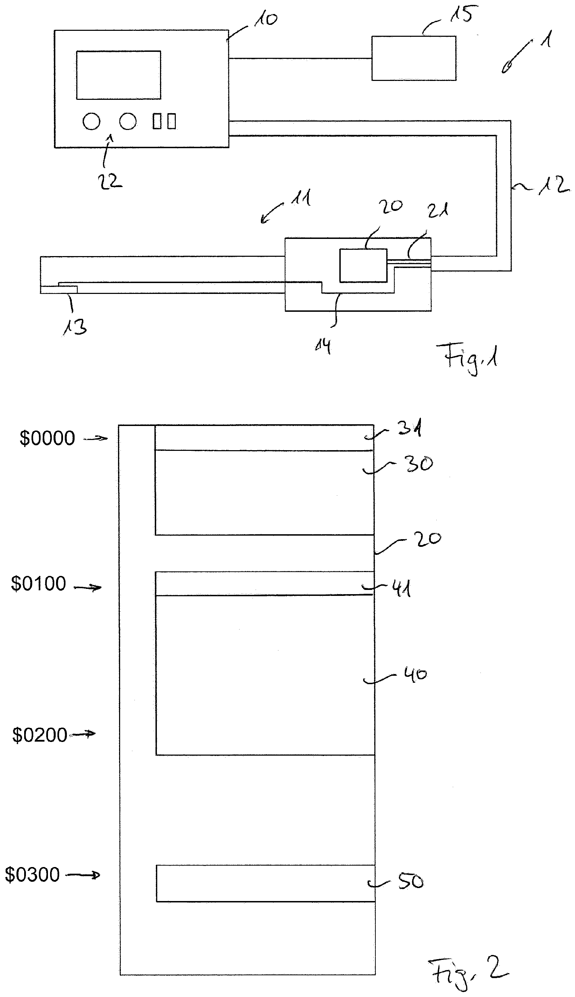

Figur 1 zeigt ein elektrochirurgisches System 1 mit einem elektrochirurgischen Versorgungsgerät 10 in Form eines Hochfrequenzgenerators, und mit einem elektrochirurgischen Instrument 11. Das elektrochirurgische Instrument 11 ist über ein Kabel 12 mit dem elektrochirurgischen Versorgungsgerät 10 verbunden. Anstelle des dargestellten Kabels 12 kann die Verbindung zwischen dem elektrochirurgischen Instrument 11 und dem Versorgungsgerät 10 auch kontaktlos, beispielsweise mit Hilfe von NFC (Near Field Communication) bzw. RFID (Radio Frequency Identification) hergestellt werden.

An einem distalen Ende des elektrochirurgischen Instruments 11 ist eine Elektrode 13 angeordnet, mit welcher Gewebe behandelt werden kann. Dazu ist die Elektrode 13 über eine Leitung 14 mit dem elektrochirurgischen Versorgungsgerät 10 verbunden.

Das elektrochirurgische Instrument kann mehr als eine Elektrode aufweisen. Alternativ oder zusätzlich zu der Elektrode 13 kann das elektrochirurgische Instrument 10 einen oder mehrere Ultraschall-Transducer umfassen.

Das elektrochirurgische Versorgungsgerät 10 erzeugt ein hochfrequentes elektrisches Signal, welches über die Leitung 14 an die Elektrode 13 geleitet wird und dort einen therapeutischen Effekt auf nicht dargestelltes Gewebe ausübt. Um den elektrochirurgischen Stromkreis zu schließen, kann eine Neutralelektrode 15 vorgesehen sein, welche ebenfalls mit dem elektrochirurgischen Versorgungsgerät 10 verbunden ist.

Das elektrochirurgische Instrument 11 ist mit einem Speicherelement 20 ausgestattet, auf welchem Konfigurationsdaten hinterlegt sind. Sobald das elektrochirurgische Instrument 11 mit dem elektrochirurgischen Versorgungsgerät 10 verbunden ist, liest das elektrochirurgische Versorgungsgerät 10 das Speicherelement 20 über Leitungen 21 aus. Das elektrochirurgische Versorgungsgerät 10 nutzt die von dem Speicherelement 20 geladenen Konfigurationsdaten, um das elektrische Signal einzustellen, welches an das elektrochirurgische Instrument 11 abgegeben wird. Dabei können verschiedene Eigenschaften des elektrischen Signals auch über Kontrollelemente 22 an dem elektrochirurgischen Versorgungsgerät 10 beeinflusst werden.

Die Konfigurationsdaten sind auf dem Speicherelement 20 in einer flexiblen Datenstruktur abgelegt, die in Figur 2 schematisch dargestellt ist. Dabei ist der logische Inhalt des Speicherelements 20 mit von oben nach unten ansteigender Speicheradresse dargestellt.

An einer ersten Speicheradresse, beispielsweise an der logischen Adresse $0000, ist ein erster Satz 30 von Konfigurationsdaten abgelegt. Ein zweiter Satz 40 von Konfigurationsdaten ist hinter dem ersten Satz 30 abgelegt, beispielsweise an der logischen Adresse $0100. Hinter dem zweiten Satz 40 von Konfigurationsdaten ist hier ein Abschlussdatenelement 50 abgelegt, beispielsweise an der logischen Adresse $0300.

Die logischen Adressen werden vorliegend zum besseren Verständnis in Hexadezimalzahlen ($...) angegeben, so entsprechen $0100 einem Wert von 256, $0200 einem Wert von 512, usw.

Der erste Satz 30 von Konfigurationsdaten kann für eine ältere Generation von elektrochirurgischen Versorgungsgeräten 10 vorgesehen sein. Solche Versorgungsgeräte erwarten nur einen einzigen Satz von Konfigurationsdaten auf dem Speicherelement 20, welcher sich immer an der logischen Adresse $0001 befindet. Dieser Satz von Konfigurationsdaten ist in seinem Inhalt eingeschränkt, da er nur Parameter für elektrochirurgische Instrumente und Signalformen enthalten kann, die bei der Entwicklung der entsprechenden Generation von elektrochirurgischen Versorgungsgeräten bereits bekannt waren.

Für neuere Instrumente oder Signalformen ist der zweite Satz 40 von Konfigurationsdaten vorgesehen. Ein modernes elektrochirurgisches Versorgungsgerät 10 ist in der Lage, Konfigurationsdaten auch von anderen logischen Adressen des Speicherelements 20 zu lesen, und kann so auf den zweiten Satz 40 von Konfigurationsdaten zugreifen.

In dem zweiten Satz 40 von Konfigurationsdaten können Konfigurationsdaten hinterlegt sein, welche die Konfigurationsdaten des ersten Satzes 30 ergänzen, so dass sie nur mit diesen Konfigurationsdaten zusammen genutzt werden können. Alternativ können die im zweiten Satz 40 hinterlegten Konfigurationsdaten in sich vollständig sein.

Die logische Adresse, an welcher der zweite Satz 40 von Konfigurationsdaten abgelegt ist, hängt von der Länge des ersten Satzes 30 von Konfigurationsdaten ab. Dabei kann die Länge des ersten Satzes 30 fest und bekannt sein, so dass die logische Adresse des zweiten Satzes 40 ebenfalls bekannt ist.

Der erste Satz 30 von Konfigurationsdaten kann auch eine variable Länge aufweisen. In diesem Fall umfasst der erste Satz 30 ein erstes Längendatenelement 31, welches die Länge das ersten Satzes 30 und/oder die Adresse des nächsten Satzes von Konfigurationsdaten anzeigt.

Der zweite Satz 40 von Konfigurationsdaten wird in der Regel immer eine variable Länge aufweisen und daher ebenfalls ein zweites Längendatenelement 41 umfassen, welches die Länge des zweiten Satzes 40 anzeigt.

Neben dem ersten Satz 30 und dem zweiten Satz 40 können beliebig viele weitere Sätze von Konfigurationsdaten in dem Speicherelement 20 abgelegt sein. Um das Ende der Datensätze anzuzeigen, ist hinter dem letzten Satz das Abschlussdatenelement 50 abgelegt.

Die einzelnen Sätze 30, 40 können direkt aneinander anschließen. In der Regel wird jedoch das Speicherelement 20 nur blockweise gelesen und/oder beschrieben werden können, beispielsweise in Blöcken mit einer Länge von $0100. Da die einzelnen Sätze 30, 40 nicht zwangsläufig ebenfalls eine Länge von $0100 haben, können zwischen einzelnen Sätzen 30, 40 bzw. dem Abschlussdatenelement 50 ungenutzte Speicherbereiche liegen.

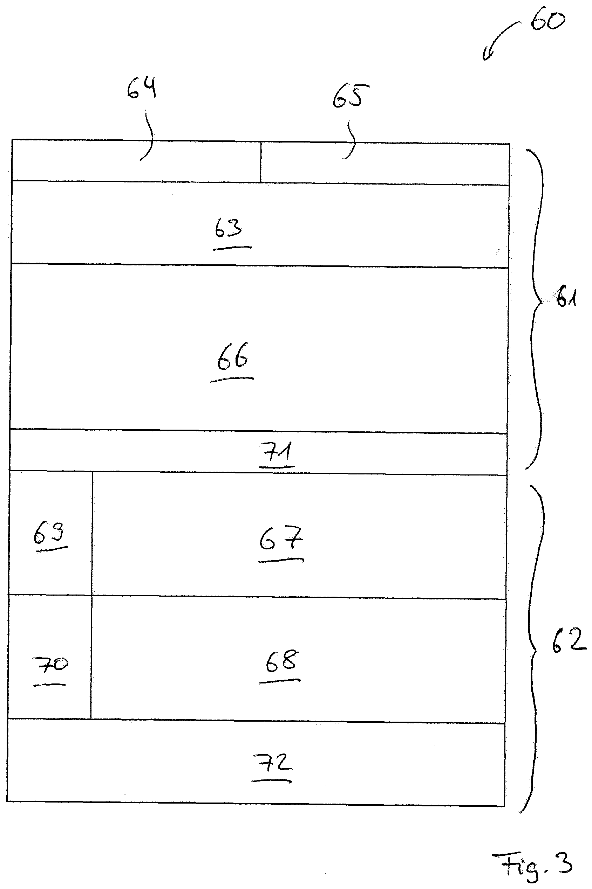

In Figur 3 ist ein möglicher Aufbau eines Satzes 60 von Konfigurationsdaten im Detail dargestellt, welcher anstelle von oder zusätzlich zu den Sätzen 30, 40 in der Datenstruktur auf dem Speicherelement 20 abgelegt sein kann.

Der Satz 60 weist zwei Abschnitte 61, 62 auf.

Der erste Abschnitt beginnt mit einem Definitionsdatenelement 63.

Das Definitionsdatenelement 63 enthält Angaben zu der Struktur des Satzes 60, d.h. Typen- und/oder Versionsbeschreibung, Längen- und/oder Postionsangaben dieses und weiterer Abschnitte innerhalb des Satzes 60, und/oder Positionsangaben des nächsten Satzes von Konfigurationsdaten, sowie spezifische Informationen des Speicherelements 20, wie beispielsweise die Blockgröße, mit der auf dem Speicherelement gelesen oder geschrieben werden kann. Dabei können Typen- und/oder Versionsdaten in einem Typdatenelement 64 enthalten sein, und Längen- und/oder Positionsangaben können in einem Längendatenelement 65 enthalten sein. Das Typdatenelement 64 und/oder das Längendatenelement 65 können eigenständige Datenelemente oder Unterelemente des Definitionsdatenelements 63 sein.

Mit Hilfe des Definitionsdatenelements 63 ist das Versorgungsgerät 10 imstande zu entscheiden, ob es mit dem Datensatz 60 kompatibel ist.

Falls der Satz 60 der letzte Satz Konfigurationsdaten in der Datenstruktur ist, kann als logische Speicheradresse des nächsten Satzes $0000 in dem Definitionsdatenelement 63 eingetragen sein.

Das Definitionsdatenelement 63 entscheidet über die Struktur des Parameterdatenelements 66 sowie des weiteren Abschnitts 62.

Das Parameterdatenelement 66 enthält die eigentlichen Konfigurationsdaten zur Bestimmung des vom Versorgungsgerät 10 abzugebenden elektrischen Signals.

Der Abschnitt 61 des Satzes 60 von Konfigurationsdaten ist als "read only" Bereich definiert, d.h. dass die in dem Abschnitt 61 hinterlegten Daten betriebsmäßig nicht durch das Versorgungsgerät 10 verändert werden können.

Der zweite Abschnitt 62 umfasst zwei Betriebsdatenelemente 67, 68. In den Betriebsdatenelementen 67, 68 werden durch das elektrochirurgische Versorgungsgerät 10 Betriebsdaten des elektrochirurgischen Instruments 11 abgelegt. Bei diesen Betriebsdaten kann es sich um Verbrauchsdaten, beispielsweise Anzahl und Dauer der Aktivierungen und/oder abgegebene Energie/Leistung des Instruments 11, Zeitstempel, Temperaturdaten oder ähnliches handeln. Die Betriebsdaten können durch das elektrochirurgische Versorgungsgerät 10 ausgewertet werden, um festzustellen, ob eine weitere Nutzung des Instruments 11 zulässig ist.

Um ein Beschreiben der Betriebsdatenelemente 67, 68 durch das Versorgungsgerät 10 zu ermöglichen, ist der Abschnitt 62 als "read / write" Bereich definiert, d.h. dass Schreibzugriffe durch das Versorgungsgerät 10 zulässig sind.

Da bei dem Beschreiben der Betriebsdatenelemente 67, 68 Fehler auftreten können, beispielsweise durch Störsignale, werden die Betriebsdatenelemente 67, 68 durch das Versorgungsgerät 10 abwechselnd beschrieben. Auf diese Weise ist sichergestellt, dass jeweils die im vorigen Schreibzugriff abgelegten Betriebsdaten noch verfügbar sind, wenn ein Schreibzugriff fehlgeschlagen ist.

Um zu ermitteln, welches der Betriebsdatenelemente 67, 68 die aktuellsten Betriebsdaten enthält, und welches als nächstes zu beschreiben ist, ist jedem der Betriebsdatenelemente 67, 68 ein Schreibflag zugeordnet 69, 70. Nach einem erfolgreichen Schreibvorgang in eines der Betriebsdatenelemente 67, 68 wird das zugeordnete Schreibflag 69 bzw. 70 getoggelt, also von "Null" auf "Eins" bzw. von "Eins" auf "Null" gesetzt.

Das Versorgungsgerät 10 liest vor jedem Lese- oder Schreibzugriff auf die Betriebsdatenelemente 67, 68 die Schreibflags 69, 70 aus. Haben beide Schreibflags 69, 70 den gleichen Wert, so sind die Betriebsdaten im Betriebsdatenelement 67 aktuell, und die nächsten Betriebsdaten sind in das Betriebsdatenelement 68 zu schreiben. Haben hingegen die Schreibflags 69, 70 unterschiedliche Werte, so sind die Betriebsdaten in dem Betriebsdatenelement 68 aktuell, und die nächsten Betriebsdaten sind in das Betriebsdatenelement 67 zu schreiben.

Im Auslieferungszustand des Instruments 11 sind die Abschnitte 67, 68, 69 und 70 mit einer vorgegebenen Zahlenfolge, beispielsweise der Fibonacci-Folge, beschrieben. Das Versorgungsgerät 10 versucht zunächst diese Zahlenfolge zu erkennen. Enthalten diese Abschnitte diese Folge, weiß das Versorgungsgerät 10, dass es sich bei dem Instrument 11 um ein unbenutztes Instrument handelt.

Nach der ersten Nutzung des Instruments 11 schreibt das Versorgungsgerät Betriebsdaten in das Betriebsdatenelement 68, und setzt das Schreibflag 70 auf den Wert "Null". Bei den Betriebsdaten kann es sich um Verbrauchsdaten, beispielsweise Anzahl und Dauer der Aktivierungen und/oder abgegebene Energie/Leistung des Instruments 11, Zeitstempel, Temperaturdaten oder ähnliches handeln.

Bei der nächsten Benutzung des Instruments 11 mit dem Versorgungsgerät 10 oder mit einem anderen Versorgungsgerät, wird nun erkannt, dass der Abschnitt 62 nur die erste Hälfte der vorgegebenen Zahlenfolge, beispielsweise der Fibonacci-Folge, enthält. Dadurch, weiß das Versorgungsgerät 10, dass die aktuellen Betriebsdaten im Betriebsdatenelement 68 abgelegt sind, und dass die nächsten Betriebsdaten in das Betriebsdatenelement 67 zu schreiben sind. Das Schreibflag 69 wird auf den Wert "Null" gesetzt.

Bei der nächsten Benutzung des Instruments 11 mit dem Versorgungsgerät 10, oder mit einem anderen Versorgungsgerät, werden die Flags 69, 70 ausgelesen. Da deren Werte nun gleich sind, weiß das Versorgungsgerät, dass die aktuellen Betriebsdaten im Betriebsdatenelement 67 abgelegt sind, und dass die nächsten Betriebsdaten in das Betriebsdatenelement 68 zu schreiben sind. Das Schreibflag 70 wird auf den Wert "Eins" gesetzt. Bei der nächsten Benutzung des Instruments 11 mit dem Versorgungsgerät 10, oder mit einem anderen Versorgungsgerät, werden wiederum die Flags 69, 70 ausgelesen. Da deren Werte nun ungleich sind, weiß das Versorgungsgerät, dass die aktuellen Betriebsdaten im Betriebsdatenelement 68 abgelegt sind, und dass die nächsten Betriebsdaten in das Betriebsdatenelement 67 zu schreiben sind.

Anstelle von zwei Schreibflags 69, 70 kann auch ein einziges Schreibflag verwendet werden, welches nach jedem Schreibvorgang getoggelt wird. Der Wert des Schreibflags gibt dann an, welches Betriebsdatenelement aktuelle Betriebsdaten enthält, und in welches Betriebsdatenelement als nächstes geschrieben werden soll.

Die Definition einzelner Bereiche des Speicherelements 20 als "read only" bzw. "read / write" ist wiederum nur für einzelne Speicherblöcke mit einer vorgegebenen Größe möglich, die nicht zwangsläufig mit den Größen der Abschnitte 61, 62 übereinstimmt. Daher können am Ende der Abschnitte 61, 62 ungenutzte Speicherbereiche 71, 72 vorhanden sein. Ähnliche, nicht dargestellte ungenutzte Speicherelemente können zwischen den einzelnen Datenelementen angeordnet sein.

Um die Integrität der in den einzelnen Datenelementen abgelegten Daten sicherzustellen, kann die Datenstruktur nicht dargestellte Prüfsummenelemente enthalten.

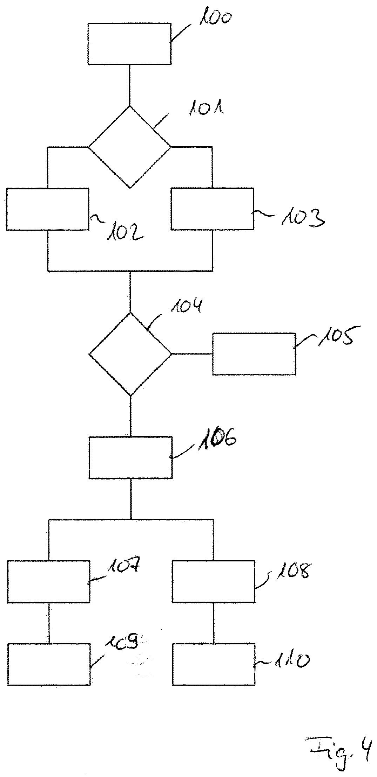

Ein Verfahren zum Lesen und Schreiben der Betriebsdaten durch ein Versorgungsgerät 10 ist in Figur 4 dargestellt. In einem ersten Schritt 100 werden die Schreibflags 69, 70 ausgelesen und in einem zweiten Schritt 101 miteinander verglichen. Haben beide Schreibflags 69, 70 den gleichen Wert, so wird in Schritt 102 das Betriebsdatenelement 67 ausgelesen. Sind die Werte der Schreibflags 69, 70 unterschiedlich, so wird in Schritt 103 das Betriebsdatenelement 68 ausgelesen.

In Schritt 104 wird anhand der ausgelesenen Betriebsdaten geprüft, ob eine weitere Nutzung des Instruments 11 zulässig ist. Ist dies nicht der Fall, so wird in Schritt 105 eine entsprechende Meldung durch das Versorgungsgerät 10 ausgegeben, und das Verfahren wird abgebrochen.

Ist die Nutzung zulässig, so wird das Instrument 11 in Schritt 106 durch das Versorgungsgerät 10 aktiviert, wobei aus dem Speicherelement 20 geladene Konfigurationsdaten und ggf. Benutzereingaben berücksichtigt werden.

Nach der Nutzung werden anhand des Ergebnisses des Vergleichs in Schritt 101 entweder in Schritt 107 aktuelle Betriebsdaten in das Betriebsdatenelement 68 geschrieben, wenn die Werte der Schreibflags 69, 70 gleich waren, oder in Schritt 108 aktuelle Betriebsdaten in das Betriebsdatenelement 67 geschrieben, wenn die Werte der Schreibflags 69, 70 unterschiedlich waren. Nach erfolgreichem Schreibvorgang wird das entsprechende Schreibflag 69 (Schritt 109) oder 70 (Schritt 110) getoggelt, damit ist das Verfahren abgeschlossen.

Für den Fall, dass die Datenstruktur mehrere Sätze von Konfigurationsdaten enthält, die beschreibbare Betriebsdatenelemente umfassen, so sollten aktuelle Betriebsdaten in jeden Satz einzeln geschrieben werden. Nur so kann sichergestellt sein, dass ein Versorgungsgerät, welches nur einen Teil der Sätze von Konfigurationsdaten ausliest, auch auf aktuelle Betriebsdaten zugreift.

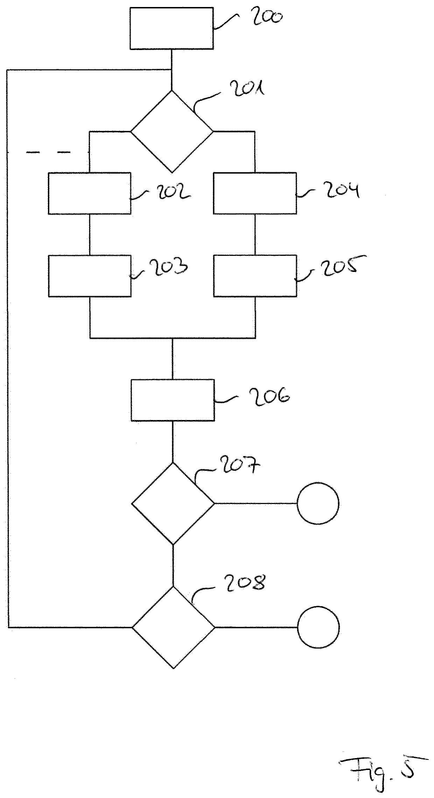

Ein Verfahren zum Auslesen der Sätze 30, 40, 60 von Betriebsdaten aus dem Speicherelement 20 durch das Versorgungsgerät 10 ist in Figur 5 dargestellt. Hierbei wird in einem ersten Schritt 200 ein erster Datenblock, beginnend bei der logischen Adresse $0000, aus dem Speicherelement ausgelesen. In Schritt 201 wird geprüft, ob der gelesene Datenblock ein Längendatenelement 31 eines ersten Satzes 30 von Konfigurationsdaten enthält. Ist dies der Fall, so wird in Schritt 202 der komplette erste Satz 30 aus dem Speicher 20 ausgelesen, und in Schritt 203 wird die logische Speicheradresse des nächsten Satzes von Konfigurationsdaten anhand des Inhalts des Längendatenelements 31 bestimmt.

Enthält der erste Datenblock kein Längendatenelement, so wird in Schritt 204 der erste Satz 30 unter der Annahme gelesen, dass dieser eine feste bekannte Länge aufweist.

Dementsprechend wird in Schritt 205 die logische Speicheradresse des nächsten Satzes von Konfigurationsdaten anhand der bekannten festen Länge des ersten Satzes 30 bestimmt.

Als nächstes wird in einem Schritt 206 ein Datenblock von der zuvor ermittelten nächsten logischen Speicheradresse gelesen, und in Schritt 207 wird geprüft, ob dieser Datenblock sinnvolle Daten enthält. Enthält der entsprechende Datenblock keine sinnvollen Daten, so sind alle Sätze aus der Speicherelement 20 ausgelesen und das Verfahren wird beendet.

Im vorliegenden Beispiel kann unter der Formulierung "enthält keine sinnvollen Daten" auch ein Fall enthalten sein, dass die ermittelte logische Speicheradresse außerhalb des zugreifbaren Speicherbereichs des Speicherelements 20 liegt, beispielsweise wenn der erste Satz 30 von Konfigurationsdaten in einem elektrochirurgischen Instrument einer älteren Generation das Speicherelement fast vollständig ausfüllt. In diesem Fall muss die Software zur Umsetzung des beschriebenen Verfahrens in der Lage sein, einen ggf. auftretenden Speicheradressfehler abzufangen.

Im Übrigen umfasst die Formulierung "enthält keine sinnvollen Daten" jeden Fall, in dem der gelesene Datenblock nicht entweder ein Typdatenelement und/oder ein Längendatenelement eines weiteren Satzes von Konfigurationsdaten, oder ein Datenabschlusselement umfasst.

In einem nächsten Schritt 208 wird dann geprüft, ob der gelesene Datenblock ein Datenabschlusselement 50 beinhaltet. In diesem Fall sind ebenfalls alle Sätze von Konfigurationsdaten ausgelesen, und das Verfahren wird beendet.

Enthält der Datenblock hingegen ein Typdatenelement und/oder ein Längendatenelement eines weiteren Satzes von Konfigurationsdaten, so wird das Verfahren ab Schritt 201 wiederholt. Die Abfrage in Schritt 201 muss in die Schleife nicht einbezogen werden, wenn bis auf den ersten Satz 30 alle weiteren Sätze von Konfigurationsdaten immer ein Längendatenelement beinhalten. In diesem Fall kann nach Schritt 208 direkt zu Schritt 202 gesprungen werden, was durch die gestrichelte Linie in Figur 5 angedeutet ist.

Nach Abschluss des Verfahrens kann das Versorgungsgerät 10 anhand der Inhalte der eingelesenen Typdatenelemente ermitteln, ob die jeweiligen Sätze mit dem Versorgungsgerät 10 kompatibel sind, und nur solche kompatiblen Sätze berücksichtigen.

## Patentansprüche

1. Elektrochirurgisches Instrument (11) zur Verwendung mit einem elektrochirurgischen System,
wobei das elektrochirurgische Instrument (11) ein Speicherelement (20) umfasst, welches durch ein elektrochirurgisches Versorgungsgerät (10) ausgelesen werden kann, wenn das elektrochirurgische Instrument (11) mit dem elektrochirurgischen Versorgungsgerät (10) verbunden ist, und
auf dem Speicherelement (20) Konfigurationsdaten hinterlegt sind, welche von dem elektrochirurgischen Versorgungsgerät (10) ausgewertet werden können, um mit dem elektrochirurgischen Instrument (11) kompatible Betriebsparameter einzustellen, **dadurch gekennzeichnet, dass** die Konfigurationsdaten in einer flexiblen Datenstruktur organisiert sind, welche es erlaubt, auf dem Speicherelement (20) gleichzeitig mehrere Sätze (30, 40, 60) von Konfigurationsdaten vorzuhalten, welche von unterschiedlichen Typen von elektrochirurgischen Versorgungsgeräten (10) ausgelesen und ausgewertet werden können, wobei
die flexible Datenstruktur einen ersten Satz (30) von Konfigurationsdaten umfasst, welcher an einer fest vorgegebenen Speicheradresse des Speicherelements (20) abgelegt ist, und dass die flexible Datenstruktur weiterhin einen zweiten Satz (40) von Konfigurationsdaten umfasst, welcher an einer zweiten Speicheradresse des Speicherelements (20) abgelegt ist, welche von der Länge der ersten Satzes (30) von Konfigurationsdaten abhängig ist; wobei ferner
ein Satz (30) von Konfigurationsdaten eine fest vorgegebene Länge aufweist, und wenigstens ein Satz (40) von Konfigurationsdaten eine flexible Länge aufweist, und ein Längendatenelement (41) umfasst, welches die Länge des entsprechenden Satzes von Konfigurationsdaten und/oder die Adresse eines nächsten Satzes von Konfigurationsdaten anzeigt.

2. Elektrochirurgisches System, umfassend:
- ein elektrochirurgisches Versorgungsgerät (10), und
- ein elektrochirurgisches Instrument nach Anspruch 1.

3. Elektrochirurgisches System nach Anspruch 2, wobei die Datenstruktur ein Abschlussdatenelement (50) umfasst, welches das Ende der Datenstruktur anzeigt.

4. Elektrochirurgisches System nach einem der Ansprüche 2-3, wobei die Datenstruktur für wenigstens einen Satz (60) von Konfigurationsdaten einen betriebsmäßig nicht überschreibbaren Speicherbereich (61) vorsieht.

5. Elektrochirurgisches System nach einem der Ansprüche 2-4, wobei die Datenstruktur für wenigstens einen Satz (60) von Konfigurationsdaten einen beschreibbaren Speicherbereich (62) vorsieht, und dass das elektrochirurgische Versorgungsgerät (10) eingerichtet ist, Betriebsdaten in dem beschreibbaren Speicherbereich (62) abzulegen.

6. Elektrochirurgisches System nach Anspruch 5, wobei der beschreibbare Speicherbereich (62) zwei Betriebsdatenelemente (67, 68) umfasst, und dass das elektrochirurgische Versorgungsgerät (10) eingerichtet ist, Betriebsdaten abwechselnd in eines der beiden Betriebsdatenelemente (6768) abzulegen.

7. Verfahren zum Auslesen von Konfigurationsdaten eines elektrochirurgischen Instruments (11) in einem elektrochirurgischen System (1) gemäß eines der Ansprüche 2 bis 6, mit den Schritten:
a) Bestimmen einer Adresse, an welcher ein erster Satz Konfigurationsdaten hinterlegt ist,
b) Einlesen eines Satzes von Konfigurationsdaten von der bestimmten Adresse,
c) Ermitteln einer Adresse, an welcher ein nächster Satz von Konfigurationsdaten erwartet wird,
d) Prüfen, ob an der in Schritt c) ermittelten Adresse ein Abschlussdatenelement (50) abgelegt ist, und
e) Wiederholen der Schritte b) bis d), bis an der ermittelten Adresse ein Abschlussdatenelement (50) festgestellt wird.

8. Verfahren nach Anspruch 7, wobei vor Schritt d) geprüft wird, ob an der ermittelten Adresse Daten hinterlegt sind, und dass das Verfahren beendet wird, wenn an der ermittelten Adresse keine Daten hinterlegt sind.

## Claims

1. An electrosurgical instrument for use with an electrosurgical system, wherein the electrosurgical instrument (11) comprises a memory element (20) that can be read by an electrosurgical power supply (10) when the electrosurgical instrument (11) is connected to the electrosurgical power supply (10), and
the memory element (20) contains configuration data that can be evaluated by the electrosurgical power supply (10) to set operating parameters compatible with the electrosurgical instrument (11),
**characterized in that** the configuration data is organized in a flexible data structure that allows multiple sets (30, 40, 60) of configuration data to be stored simultaneously on the memory element (20), which can be read and evaluated by different types of electrosurgical power supply units (10), wherein
the flexible data structure comprises a first set (30) of configuration data, which is stored at a fixed memory address of the memory element (20), and that the flexible data structure further comprises a second set (40) of configuration data, which is stored at a second memory address of the memory element (20) that depends on the length of the first set (30) of configuration data; wherein, further,
one set (30) of configuration data has a fixed length, and
at least one set (40) of configuration data has a flexible length and comprises a length data element (41) that indicates the length of the corresponding set (40) of configuration data and/or the address of a subsequent set of configuration data.

2. An electrosurgical system comprising:
- an electrosurgical power supply (10), and
- an electrosurgical instrument (11) according to claim 1.

3. An electrosurgical system according to claim 2, wherein the data structure comprises a terminator data element (50) that indicates the end of the data structure.

4. An electrosurgical system according to any one of claims 2-3, wherein the data structure provides a memory area (61) for at least one set (60) of configuration data that cannot be overwritten during operation.

5. An electrosurgical system according to any one of claims 2-4, wherein the data structure provides a writable memory area (62) for at least one set (60) of configuration data, and wherein the electrosurgical power supply (10) is configured to store operating data in the writable memory area (62).

6. An electrosurgical system according to claim 5, wherein the writable memory area (62) comprises two operational data elements (67, 68), and wherein the electrosurgical power supply unit (10) is configured to store operational data alternately in one of the two operational data elements (67, 68).

7. A method for reading configuration data from an electrosurgical instrument (11) in an electrosurgical system (1) according to any one of claims 2 through 6, comprising the steps of:
a) determining an address at which a first set of configuration data is stored,
b) reading a set of configuration data from the determined address,
c) determining an address at which a subsequent set of configuration data is expected,
d) checking whether a terminating data element (50) is stored at the address determined in step c), and
e) repeating steps b) through d) until a terminating data element (50) is detected at the determined address.

8. The method according to claim 7, wherein, prior to step d), a check is performed to determine whether data is stored at the determined address, and the method is terminated if no data is stored at the determined address.

## Revendications

1. Instrument électrochirurgical destiné à être utilisé avec un système électrochirurgical, l'instrument électrochirurgical (11) comprenant un élément de mémoire (20) pouvant être lu par un appareil d'alimentation électrochirurgical (10) lorsque l'instrument électrochirurgical (11) est connecté à l'appareil d'alimentation électrochirurgical (10), et
sur lequel élément de mémoire (20) sont stockées des données de configuration pouvant être analysées par l'appareil d'alimentation électrochirurgical (10) afin de régler des paramètres de fonctionnement compatibles avec l'instrument électrochirurgical (11),
**caractérisé en ce que** les données de configuration sont organisées selon une structure de données flexible qui permet de stocker simultanément plusieurs ensembles (30, 40, 60) de données de configuration sur l'élément de mémoire (20), lesquelles peuvent être lues et analysées par différents types d'appareils d'alimentation électrochirurgicaux (10),
la structure de données flexible comprenant un premier ensemble (30) de données de configuration, qui est stocké à une adresse mémoire prédéfinie de l'élément de mémoire (20), et **en ce que** la structure de données flexible comprend en outre un deuxième ensemble (40) de données de configuration, qui est stocké à une deuxième adresse mémoire de l'élément de mémoire (20), laquelle dépend de la longueur du premier ensemble (30) de données de configuration;
dans lequel, en outre, un ensemble (30) de données de configuration présente une longueur prédéfinie, et
au moins un ensemble (40) de données de configuration présente une longueur flexible et comprend un élément de données de longueur (41) qui indique la longueur de l'ensemble (40) de données de configuration correspondant et/ou l'adresse d'un ensemble suivant de données de configuration.

2. Système électrochirurgical comprenant :
- un appareil d'alimentation électrochirurgical (10), et
- un instrument électrochirurgical (11) selon la revendication 1.

3. Système électrochirurgical selon la revendication 2, dans lequel la structure de données comprend un élément de données de fin (50) qui indique la fin de la structure de données.

4. Système électrochirurgical selon l'une des revendications 2 à 3, dans lequel la structure de données prévoit, pour au moins un ensemble (60) de données de configuration, une zone de mémoire (61) qui ne peut pas être écrasée en cours de fonctionnement.

5. Système électrochirurgical selon l'une des revendications 2 à 4, dans lequel la structure de données prévoit, pour au moins un ensemble (60) de données de configuration, une zone de mémoire inscriptible (62), et dans lequel l'appareil d'alimentation électrochirurgical (10) est agencé pour stocker des données de fonctionnement dans la zone de mémoire inscriptible (62).

6. Système électrochirurgical selon la revendication 5, dans lequel la zone de mémoire inscriptible (62) comprend deux éléments de données de fonctionnement (67, 68), et dans lequel l'appareil d'alimentation électrochirurgical (10) est agencé pour stocker des données de fonctionnement alternativement dans l'un des deux éléments de données de fonctionnement (67, 68).

7. Procédé de lecture de données de configuration d'un instrument électrochirurgical (11) dans un système électrochirurgical (1) selon l'une des revendications 2 à 6, comprenant les étapes suivantes :
a) déterminer une adresse à laquelle un premier ensemble de données de configuration est stocké,
b) lire un ensemble de données de configuration à partir de l'adresse déterminée,
c) déterminer une adresse à laquelle un ensemble suivant de données de configuration est attendu,
d) vérifier si un élément de données de fin (50) est stocké à l'adresse déterminée à l'étape c), et
e) répéter les étapes b) à d) jusqu'à ce qu'un élément de données de fin (50) soit détecté à l'adresse déterminée.

8. Procédé selon la revendication 7, dans lequel, avant l'étape d), on vérifie si des données sont stockées à l'adresse déterminée, et le procédé est terminé si aucune donnée n'est stockée à l'adresse déterminée.
